# EUROPÄISCHE PATENTSCHRIFT

(11) **EP 0 393 459 B1**
(45) Veröffentlichungstag und Bekanntmachung des Hinweises auf die Patenterteilung: **27.12.1995**
(21) Anmeldenummer: 90106754.6
(22) Anmeldetag: 09.04.1990
(51) Int. Cl.: G01N 33/04, G01N 21/35

(54) **Verfahren zur Erstellung von Grundeichungen von Milchprodukten, zur Ermittlung der Eigenschaften eines Milchprodukts und zur Verarbeitung eines Ausgangssubstrats**
Method for preparing calibrations of milk products for determining the properties of a milk product and for processing a basic material
Procédé d'établissement de calibrages de produits laitiers pour déterminer les propriétés d'un produit laitier et pour traiter un substrat de base

(30) Priorität: 10.04.1989 DE 3911710; 10.07.1989 DE 3922670
(43) Veröffentlichungstag der Anmeldung: 24.10.1990
(73) Patentinhaber: Milchwirtschaftliche Förderungsgesellschaft mbH Niedersachsen, D-30175 Hannover (DE)
(72) Erfinder: Wüst, Eberhard, Prof. Dr., D-3016 Seelze 6 (DE); Osmers, Karl, D-3000 Hannover 51 (DE); Rudzik, Lutz, Dr., D-3000 Hannover 91 (DE)
(74) Vertreter: Zinnecker, Armin, Dipl.-Ing.

(56) Entgegenhaltungen:
- EP-A- 0 038 254
- DE-A- 2 839 750
- FR-A- 2 087 203
- GB-A- 2 028 498
- US-A- 4 447 725
- ANALYTICAL CHEMISTRY, Band 55, Nr. 12, Oktober 1983; D.L. WETZEL, S. 1165A-1176A;
- ANALYTICAL CHEMISTRY, Band 59, Nr. 17, September 1987; P. ROBERT et al.; S. 2187-2191;

## Beschreibung

Die Erfindung betrifft ein Verfahren zur Qualitätssicherung von Milch oder Milchprodukten.

Aus der Veröffentlichung "Multivariate Analysis Applied to Near-Infrared Spectra of Milk", Anal. Chem. 59 (17) (1987), Sn. 2187 - 2191 ist es bekannt, Proben von Milch spektroskopisch zu untersuchen. Nach dieser Veröffentlichung wird eine Mehrzahl von Milchproben spektroskopisch untersucht. Die erhaltenen Spektren werden einem mathematischen Mittelungsverfahren unterworfen. Dabei werden der Mittelwert und die Varianz ermittelt.

Aus der FR 2 087 203 ist es bekannt, Butter spektroskopisch zu untersuchen und die dabei erhaltenen Meßwerte für den Herstellungsprozeß der Butter zu verwenden.

Aufgabe der Erfindung ist es, ein verbessertes und optimiertes Verfahren zu Qualitätssicherung von Milch oder Milchprodukten anzugeben.

Erfindungsgemäß wird diese Aufgabe durch die Kombination der Merkmale des Anspruchs 1 gelöst.

Unter einem Milchprodukt im Sinne der Erfindung ist dabei auch ein Zwischenprodukt zu verstehen, das bei der Herstellung von Milchprodukten anfällt. Bei den Milchprodukten handelt es sich beispielsweise um Rohmilch, Trinkmilch, Magermilch, Sahne, Kaffeesahne, Speisequark (40 %), Speisequark (20 %), Magerquark, Fruchtquark, Kondensmilch, Vollmilchpulver, Kakaogetränkepulver, Molke, molkeähnliche Substrate, Kesselmilch, Buttermilch oder ähnliches.

Milchprodukte werden in Herstellungsbetrieben (Molkereien) hergestellt und/oder weiterverarbeitet. Das den Herstellungsbetrieb verlassende Endprodukt und auch die jeweiligen Zwischenprodukte sollen bestimmte Eigenschaften aufweisen. Diejenigen Eigenschaften, die ein gewisses Milchprodukt besitzen soll, können in einem Standardsprektrum ("Grundeichung") zusammengefaßt werden.

Die in der Zentrale vorhandenen Standardspektren können allgemeine Gültigkeit besitzen. Sie können nicht nur zur Qualitätssicherung für das Produkt eines bestimmten Herstellungsbetriebes angewendet werden, sondern auch bei einem Produkt gleicher Art eines anderen Herstellungsbetriebes. Die Standardspektren können zur Qualitätssicherung und Optimierung der hergestellten Produkte sowohl in quantitativer als auch in qualitativer Hinsicht dienen.

Die Qualitätssicherung kann sich auf bestimmte Eigenschaften der Milch oder des Milchprodukts beziehen. Bei diesen Eigenschaften des Milchprodukts kann es sich um quantitative oder qualitative Eigenschaften handeln. Zur Ermittlung der qualitativen Eigenschaften ist es erforderlich, ein Spektroskopieverfahren durchzuführen, das eine ausreichende Menge von Informationen liefert. Besonders geeignet ist die registrierende Spektroskopie.

Nach der Erfindung können ein oder mehrere Ausgangssubstrate durch Schwingungsspektroskopie gemessen werden. Das Meßergebnis kann mit einem Sollwert verglichen werden. In Abhängigkeit von diesem Soll-Ist-Vergleich kann der Verarbeitungsprozeß des Ausgangssubstrats gesteuert werden. Durch die Messung durch Schwingungsspektroskopie werden das oder die Ausgangssubstrate charkterisiert. Ein Ausgangssubstrat in diesem Sinne kann auch ein Zwischenprodukt des Verarbeitungsprozesses sein. Es ist auch möglich, das Verfahren bei mehreren Ausgangssubstraten und/oder Zwischenprodukten des Verarbeitungsprozesses durchzuführen. Das Meßergebnis der Schwingungsspektroskopie kann vor dem Vergleich mit dem Sollwert verarbeitet werden. Die Steuerung in Abhängigkeit von dem Soll-Ist-Vergleich führt zu einer Einflußnahme auf den Produktionsprozeß (Verarbeitungsprozeß).

Bei dem Soll-Ist-Vergleich kann die Korrelation zwischen dem Ist-Wert (Ist-Spektrum) und dem Soll-Wert (Soll-Spektrum) bestimmt werden.

Es ist möglich, die Messung und damit auch die Charakterisierung einmal durchzuführen. Bereits hierdurch kann eine erhebliche Steigerung der Qualität des Endprodukts erreicht werden. Es ist aber auch möglich, die Messung und damit auch die Charakterisierung mehrmals (diskontinuierlich oder kontinuierlich) durchzuführen. Die zu messenden und nachzuführenden Eigenschaften können sensorische, chemische und/oder physikalische Eigenschaften sein. Es ist auch möglich, die Messung (Charakterisierung) vor und nach einem bestimmten Verarbeitungsschritt durchzuführen, beispielsweise vor und nach der Erhitzung. Die Messung findet vorzugsweise unter standardisierten Bedingungen statt. Beispielsweise können die Temperatur des Meßgutes und/oder die Teilchengröße des Meßgutes genau standardisiert sein, um reproduzierbare Ergebnisse erzielen zu können.

Die Steuerung kann durch verfahrenstechnische Eingriffe erfolgen, beispielsweise dadurch, daß in einem Separator dessen Geschwindigkeit, Stellung der Seitbleche, Temperatur, Zudosierung etc. verändert wird.

Vorteilhafte Weiterbildungen der Erfindung sind in den Unteransprüchen beschrieben.

Als spektroskopisches Untersuchungsverfahren kann die sogenannte MIR-Spektroskopie verwendet werden, also die Spektroskopie im mittleren Infrarotbereich (5.000-180 1/cm). Die MIR-Spektroskopie ist z.B. zur quantitativen Bestimmung der Milchinhaltsstoffe in Milch und flüssigen Milchprodukten im Einsatz (Milko-Scan- und Multispec-Systeme).

Auch die Verwendung der NIR-Spektroskopie, also der Spektroskopie im nahen Infrarotbereich (12.500-5.000 1/cm) ist möglich. Die NIR-Spektroskopie kann auch zu quantitativen Messungen der Inhaltsstoffe fester und pastöser Proben eingesetzt werden, wobei hier besonders die diffuse Reflexion des IR-Lichtes als Meßparameter ausgenutzt wird. Hierbei können Filtergeräte zum Einsatz kommen, also Geräte, die nur einige wenige - wenn auch signifikante - Wellenzahlbereiche des gesamten Infrarotspektrums messen. Ein Filtergerät mißt also nur an bestimmten, ausgewählten Wellenlängenintervallen eines Spektrums.

Darüberhinaus ist es von Vorteil, ein registrierendes Gerät einzusetzen, also ein Gerät, welches das gesamte Spektrum in einem bestimmten Infrarotbereich aufzeichnet. Die registrierende IR- Spektroskopie gibt über die quantitative Analyse hinaus die Möglichkeit, qualitative Untersuchungen an Milch und Milchprodukten und auch an Zwischenprodukten durchzuführen.

Zunächst wird die Probe durch gezielte Maßnahmen der Probenvorbereitung (z.B. Temperierung, Zerkleinerung auf eine bestimmte Teilchengröße, Einfüllen in das Probegefäß unter Berücksichtigung einer definierten Packungsdichte usw.) für die Messung vorbereitet. Nach der eigentlichen Messung erfolgt eine Daten- bzw. Spektrenauswahl. Unter Zuhilfenahme von mathematischen Algorithmen wird dann von diesen Daten bzw. Spektren eine Grundeichung (Kalibrierung) erstellt. Danach schließt sich eine Umsetzung der Eichung an. Dabei kommen spezielle Algorithmen zur Anwendung. Aus der Grundeichung entsteht so eine modifizierte Eichung, die allgemeingültig ist. Diese Kalibrierungen (Eichungen) können dann per Datentransfer in die Satellitensysteme übertragen werden und dort z.B. zur Prozeßsteuerung dienen.

Als Auswertungsverfahren sind mathematische Mittelungsverfahren geeignet. Ebenfalls geeignet ist das sogenannte FSD-Verfahren (Fourier-self-deconvolution-Verfahren). Beim FSD-Verfahren handelt es sich um einen mathematischen Algorithmus, der eine Entfaltung der Spektren ermöglicht. Viele technologische Verfahrensschritte äußern sich durch FSD-Entzerrung der Eiweißbanden auf charakteristische Weise. Dadurch lassen sich bestimmte "Qualitäten" der Milch und Milchprodukte beschreiben, eichen und sichern.

Die NIR-Analytik bietet einen wesentlich höheren Informationsgehalt.

Mit der Anzahl der untersuchten Proben steigt die Zuverlässigkeit und Genauigkeit der damit erstellten Kalibrierung. Durch die Spektroskopie, insbesondere durch die registrierende Spektroskopie, können auch Eigenschaften an Produkten charakterisiert und einkalibriert werden, die über unmittelbare physikalische und/oder chemische Eigenschaften hinausgehen, insbesondere solche Eigenschaften, die ihre Grundlage in einer menschlichen Bewertung haben. Als Beispiele sind zu nennen sensorische Eigenschaften, das Koagulationsverhalten von beispielsweise Kaffeesahne, die Verdaulichkeit, der Aschegehalt und ähnliches.

Die Kalibrierungen können nicht nur für die verschiedenen Milchprodukte erstellt werden, sondern auch für ein bestimmtes Milchprodukt unter jeweils verschiedenen Bedingungen. So ist es beispielsweise möglich, Milch vor und nach der UHT-Erhitzung, abhängig von verschiedenen Tankeinstellungen und abhängig von der Sommer- und Winterfütterung zu kalibrieren.

Die Erstellung von Kalibrierungen kann zentral zusammengefaßt werden. Es ist möglich, in den einzelnen Molkereien eine Spektroskopie beispielsweise durch NIR-Filtergeräte vorzunehmen. Die Ergebnisse werden dann per Datenfernübertragung, beispielsweise durch ein Telefonmodem, an das zentrale Muttersystem übermittelt. Das Muttersystem erstellt die für die Arbeit an den Produktionslinien in den jeweiligen Molkereien nötigen Kalibrierungen und überträgt die fertigen Kalibrierungen in die NIR-Geräte in den Molkereien. Dort werden die Kalibrierungen dann verwendet. Auf diese Weise ist eine "Online-Kalibrierungspflege" möglich.

Bei der Spektroskopie handelt es sich um die sogenannte Schwingungsspektroskopie. Es ist möglich, die beiden Arten der Schwingungsspektroskopie durchzuführen, also die Infrarot-Schwingungsspektroskopie und die Raman-Spektroskopie. Letztere beruht auf einer Spektroskopie des Streulichts; sie hat den besonderen Vorteil, daß Dipolschwingungen nicht gemessen, also nicht erfaßt werden. Dies wirkt sich insbesondere dahingehend aus, daß das in der Probe enthaltene Wasser nicht erfaßt, also nicht "gesehen" wird. Es ist möglich, Transmissions-Spektroskopie oder Reflexions-Spektroskopie anzuwenden.

Die Kalibrierung kann auch so durchgeführt werden, daß mit Hilfe eines Rechenverfahrens die repräsentativen Proben ermittelt werden. Das Rechenverfahren kann dem Gauss-Jordan-Algorithmus ähneln, also einer mathematischen Methode, mit der Gleichungssysteme über Matritzenrechnung gelöst werden können. Hierdurch werden folgende Vorteile erreicht: Die Anzahl der aufwendigen Referenzanalysen wird minimiert; die Interkorrelation zwischen den Inhaltsstoffen wird verringert. Im Gesamtergebnis wird die Erstellung der Kalibrierungen billiger und in der Qualität besser.

Nach der Erfindung wird eine Probe des Milchprodukts spektroskopisch untersucht und wird das Spektrum mit einem vorher erstellten Grundeichungs-Spektrum bzw. mit einer vorher erstellten Kalibrierung ausgewertet bzw. verglichen wird. Die Probe des Milchprodukts kann nach jeder der oben beschriebenen Spektroskopie-Arten untersucht werden.

Bei den Eigenschaften des Milchprodukts kann es sich um quantitative oder qualitative Eigenschaften handeln. Zur Ermittlung der qualitativen Eigenschaften ist es erforderlich, ein Spektroskopieverfahren durchzuführen, das eine ausreichende Menge von Informationen liefert. Besonders geeignet ist die registrierende Spektroskopie. Hinsichtlich der quantitativen und qualitativen Eigenschaften wird auf die obigen Ausführungen verwiesen.

Es ist auch möglich, daß die spektroskopische Untersuchung in der Molkerei erfolgt. Die so ermittelten Daten werden per Datenfernübertragung in die Zentrale weitergeleitet. In der Molkerei kann beispielsweis eine sichere und präzise NIR-Analytik durchgeführt werden. Deren Ergebnisse werden durch "quasi-on-line-Messungen" in die Zentrale übermittelt. Die Anpassung der Kalibrierung erfolgt dann in der Zentrale. Das Ergebnis kann wiederum durch Datenfernübertragung in die einzelne Molkerei übermittelt werden. Es kann dort zur Steuerung des Produktionsprozesses verwendet werden. Da die Spektroskopie ein verhältnismäßig schnelles Verfahren ist, sind die durch das soeben beschriebene Verfahren hervorgerufenen Zeitverzögerungen im Verhältnis zum Ablauf des Produktionsprozesses in der Molkerei sehr gering. Hierdurch wird sichergestellt, daß das von der Zentrale erstellte Ergebnis noch im selben Produktionsprozeß in der Molkerei verwendet werden kann. Dieses Verfahren dient also der exakten Qualitätssicherung. Es ist auch möglich, auf der Grundlage dieses Verfahrens ein CIM-Konzept zu realisieren (CIM = Computer-integrated-manufacturing).

Das System kann derart aufgebaut sein, daß in der Zentrale ein registrierendes NIR-Gerät vorhanden ist, das vorzugsweise mit Computerunterstützung arbeitet (Muttersystem). In den einzelnen Molkereien sind NIR-Filtergeräte vorhanden (Satellitensysteme). Die Satellitensysteme werden mit dem Muttersystem über Datenfernübertragung vernetzt; dies geschieht vorzugsweise durch ein Telefonmodem. Es ist auch möglich, daß die Satellitensysteme untereinander kommunizieren können. Durch dieses System kann den Molkereien die verhältnismäßig aufwendige naßchemische Referenzanalytik abgenommen werden. Diese wird lediglich in der Zentrale durchgeführt. Verbindet man dieses System mit dem oben beschriebenen Eichverfahren, wird der weitere Vorteil erreicht, daß die einzelnen Molkereien ihre Filtergeräte nicht selbst zu eichen brauchen. Die Eichung wird vielmehr von der Zentrale durchgeführt und den Molkereien zur Verfügung gestellt.

Die Untersuchung durch Spektroskopie, insbesondere NIR-Spektroskopie, bietet folgende Vorteile: Die NIR-Messungen sind sehr schnell; die Meßzeit beträgt etwa eine Minute. Es kann also jederzeit in den laufenden Herstellungsprozeß eingegriffen und das Verfahren optimiert werden. Daraus resultieren beträchtliche wirtschaftliche Vorteile. Weiterhin können die Analyseautomaten, die die NIR-Messungen durchführen, nach erfolgter Kalibration durch den Maschinenführer oder durch Hilfspersonal bedient werden, also durch weniger qualifiziertes Personal. Es ist möglich, simultan mehrere Inhaltsstoffe gleichzeitig sicher zu bestimmen. Umweltschädigende Stoffe (Lösungsmittel, Schwefelsäure usw.) müssen nicht eingesetzt und daher auch nicht entsorgt werden. Die Qualität der Produkte sowie die Produktionskosten werden positiv beeinflußt (Qualitätssicherung). Es kann auch Laborpersonal eingespart werden. Zum Teil werden höhere Analysegenauigkeiten erreicht als mit den bereits bekannten Standardmethoden wie z.B. der Trocknung. Darüberhinaus ist es möglich, die Analyse simultan zum Trockenprozeß ablaufen zu lassen.

Nach der Erfindung werden ein oder mehrere Ausgangssubstrate durch Schwingungsspektroskopie gemessen, wird das Meßergebnis mit einem Sollwert verglichen und wird in Abhängigkeit von diesem Soll-Ist-Vergleich der Verarbeitungsprozeß des Ausgangssubstrats gesteuert. Durch die Messung durch Schwingungsspektroskopie werden das oder die Ausgangssubstrate charakterisiert. Ein Ausgangssubstrat in diesem Sinne kann auch ein Zwischenprodukt des Verarbeitungsprozesses sein. Es ist auch möglich, das Verfahren bei mehreren Ausgangssubstraten und/oder Zwischenprodukten des Verarbeitungsprozesses durchzuführen. Die Schwingungsspektroskopie erfolgt nach einer der eingangs angegebenen Arten. Das Meßergebnis kann vor dem Vergleich mit dem Sollwert verarbeitet werden. Die Steuerung in Abhängigkeit von dem Soll-Ist-Vergleich, also in Abhängigkeit von der Charakterisierung, führt zu einer Einflußnahme auf den Verarbeitungsprozeß, also Produktionsprozeß.

Bei dem Soll-Ist-Vergleich kann die Korrelation zwischen dem Ist-Wert (Ist-Spektrum) und dem Soll-Wert (Soll-Spektrum) bestimmt werden.

Es ist möglich, die Messung und damit auch die Charakterisierung einmal durchzuführen. Bereits hierdurch kann eine erhebliche Steigerung der Qualität des Endprodukts erreicht werden. Es ist aber auch möglich, die Messung und damit auch die Charakterisierung mehrmals (diskontinuierlich oder kontinuierlich) durchzuführen.

Die zu messenden und nachzuführenden Eigenschaften können sensorische, chemische und/oder physikalische Eigenschaften sein.

Es ist auch möglich, die Charakterisierung vor und nach einem bestimmten Verarbeitungsschritt durchzuführen. Beispielsweise kann die Charakterisierung vor und nach der Erhitzung durchgeführt werden.

Die Messung bzw. Charakterisierung ist vorzugsweise standardisiert. In bestimmten Anwendungsfällen kann es erforderlich sein, die Temperatur des Meßgutes und/oder die Teilchengröße des Meßgutes genau zu standardisieren, um reproduzierbare Ergebnisse erzielen zu können.

Eine vorteilhafte Weiterbildung des erfindungsgemäßen Verfahrens ist dadurch gekennzeichnet, daß in Gesamt- oder Teilströmen quantitative und/oder qualitative Eigenschaften der Inhaltsstoffe spektroskopisch gemessen werden und daß die gemessenen Ist-Werte mit vorgegebenen Soll-Werten verglichen werden, so daß Zuordnungen zum optimalen Produkt getroffen werden können. Durch Änderungen von Inhaltsstoff-Zuordnungen können Qualitätssteigerungen erzielt werden.

Vorteilhaft ist es, wenn die Messungen mit Sollkurven verglichen werden, die bezüglich der quantitativen und/oder qualitativen Eigenschaften vorher ermittelt wurden, und wenn die Meßwerte an die Sollkurven angenähert werden.

Eine Inhaltsstoff-Zuordnung in diesem Sinne ist die Konzentration eines Inhaltsstoffs im Verhältnis zu einem anderen Inhaltsstoff oder zu mehreren anderen Inhaltsstoffen. Die Zuordnungen können quantitativ oder qualitativ oder quantitativ und qualitativ sein. Die Steuerung erfolgt durch verfahrenstechnische Eingriffe. Möglich ist es beispielsweise, einen Seperator zu steuern, also dessen Geschwindigkeit, Stellung der Leitbleche, Temperatur, Zudosierung etc. zu verändern.

## Patentansprüche

1. Verfahren zur Qualitätssicherung von Milch oder Milchprodukten, bei dem
a. eine Mehrzahl von Proben der Milch oder des jeweiligen Milchprodukts spektroskopisch untersucht und die erhaltenen Spektren durch ein mathematisches Mittelungsverfahren zu einem Standardspektrum zusammengefaßt werden,
b. im Rahmen eines Herstellungsprozesses für Milch oder Milchprodukte in mehreren Herstellungsbetrieben jeweils eine Probe der Milch oder des Milchproduktes spektroskopisch untersucht werden,
c. die so erhaltenen Spektren über Datenfernübertragung an eine Zentrale weitergeleitet werden,
d. die so übermittelten Spektren in der Zentrale mit dem Standardspektrum verglichen werden
e. und die Ergebnisse dieser Vergleiche durch Datenfernübertragung an die Herstellungsbetriebe zur Steuerung der Produktionsprozesse übermittelt werden.

2. Verfahren nach Anspruch 1, dadurch gekennzeichnet, daß die Proben mit Spektroskopie im mittleren Infrarotbereich (MIR-Spektroskopie) untersucht werden.

3. Verfahren nach Anspruch 1 oder 2, dadurch gekennzeichnet, daß die Proben mit Spektroskopie im nahen Infrarotbereich (NIR-Spektroskopie) untersucht werden.

4. Verfahren nach einem der vorhergehenden Ansprüche, dadurch gekennzeichnet, daß die Proben mit Filter-Spektroskopie untersucht werden.

5. Verfahren nach einem der vorhergehenden Ansprüche, dadurch gekennzeichnet, daß die Proben mit registrierender Spektroskopie untersucht werden.

6. Verfahren nach einem der vorhergehenden Ansprüche, dadurch gekennzeichnet, daß das Mittelungsverfahren Fourier-selfdeconvolution (FSD) und/oder Differenzspektroskopie ist.

## Claims

1. Method for quality control of milk or milk products, in which method
a. a plurality of samples of the milk or of the particular milk product are spectroscopically examined and the spectra obtained are combined by means of a mathematical averaging process into a standard spectrum,
b. within the framework of a production process for milk or milk products in a plurality of production operations, one sample of the milk or of the milk product is spectroscopically examined in each case,
c. the spectra thus obtained are forwarded via remote data transmission to a central unit,
d. the spectra thus transmitted are compared in the central unit with the standard spectrum
e. and the results of those comparisons are transmitted by means of remote data transmission to the production operations to control the production processes.

2. Method according to Claim 1, characterized in that the samples are examined using spectroscopy in the medium infrared range (MIR spectroscopy).

3. Method according to Claim 1 or 2, characterized in that the samples are examined using spectroscopy in the near infrared range (NIR spectroscopy).

4. Method according to one of the preceding claims, characterized in that the samples are examined using filter spectroscopy.

5. Method according to one of the preceding claims, characterized in that the samples are examined using recording spectroscopy.

6. Method according to one of the preceding claims, characterized in that the averaging method is Fourier self-deconvolution (FSD) and/or differential spectroscopy.

## Revendications

1. Procédé pour l'assurance de qualité du lait ou de produits laitiers dans lequel
a) un certain nombre d'échantillons du lait ou de produit laitier est examiné au spectroscope et les spectres obtenus sont rassemblés en un spectre standard par un procédé mathématique de formation de la moyenne,
b) dans le cadre d'un procédé de fabrication du lait ou de produits laitiers dans plusieurs exploitations de fabrication, à chaque fois un échantillon du lait ou du produit laitier est examiné au spectroscope,
c) les spectres ainsi obtenus sont transmis à une centrale par transmission à distance de données,
d) les spectres ainsi transmis à la centrale sont comparés au spectre standard,
e) et les résultats de cette comparaison sont transmis par transmission de données à distance à l'exploitation de fabrication pour le contrôle du procédé de production.

2. Procédé selon la revendication 1 caractérisé en ce que les échantillons sont examinés par spectroscopie en infrarouges moyens (spectroscopie MIR).

3. Procédé selon la revendication 1 ou 2 caractérisé en ce que les échantillons sont examinés par spectroscopie en infrarouges proches (spectroscopie NIR).

4. Procédé selon l'une quelconque des revendications précédentes caractérisé en ce que les échantillons sont examinés par spectroscopie à filtre.

5. Procédé selon l'une quelconque des revendications précédentes caractérisé en ce que les échantillons sont examinés par spectroscopie enregistreuse.

6. Procédé selon l'une quelconque des revendications précédentes caractérisé en ce que le procédé de formation de la moyenne est une auto déconvolution de Fourier (FSD) et/ou une spectroscopie différentielle.
